# EUROPEAN PATENT APPLICATION

(11) **EP 1 541 016 A1**
(43) Date of publication of application: **15.06.2005**
(21) Application number: 03764204.8
(22) Date of filing: 16.07.2003
(51) Int. Cl.: A01H 5/00, C12N 15/00

(54) **METHOD OF CONSTRUCTING CAFFEINELESS COFFEE PLANT BY GENETIC RECOMBINATION**

(30) Priority: 16.07.2002 JP 2002207221
(71) Applicant: Nara Institute of Science and Technology, Ikoma-shi, Nara-ken 630-0192 (JP)
(72) Inventor: OGITA, Shinjiro Toyama Kenritsu Daigaku Taikouyama, Imizu-gun, Toyama 939-0362 (JP); SANO, Hiroshi, Ikoma-shi, Nara 630-0114 (JP); KOIZUMI, Nozomu, Ikoma-shi, Nara 630-0101 (JP); SHINMYO, Atsuhiko, Ikoma-gun, Nara 636-0151 (JP)
(74) Representative: TER MEER STEINMEISTER & PARTNER GbR
(86) International application number: PCT/JP2003/009008
(87) International publication number: WO 2004/006658

(57) **Abstract**

The invention provides a process for producing a decaffeinated coffee plant by genetic recombination comprising a step for preparing an antisense sequence or an RNAi sequence of a gene coding for an enzyme related to the caffeine biosynthetic pathway and constructing an expression vector for transformation, a step for introducing the obtained expression vector into Agrobacterium, a step for infecting a cell division-activated tissue piece of a coffee plant or a callus or an adventitious embryo induced from a tissue piece of a coffee plant with the Agrobacterium to transform the tissue piece, the callus or the adventitious embryo and a step for obtaining an transformed coffee plant from a transformed tissue piece, a transformed callus or a transformed adventitious embryo so that a coffee plant with low caffeine content can be produced by suppressing expression of a gene coding for an enzyme related to the caffeine biosynthetic pathway by the antisense method or the RNAi method.

## Description

### TECHNICAL FIELD

The present invention relates to a process for producing a decaffeinated coffee plant or a caffeine-reduced coffee plant by genetic recombination.

### BACKGROUND ART

Caffeine contained in coffee has effects such as awakening function or enhancement of heart function. On the other hand, it has side effects such as sleeplessness, palpitation and dizziness. Therefore, there is a high demand for decaffeinated coffee and it was produced mainly by using a physicochemical isolation method such as organic solvent extraction conventionally. However, this method results in high production cost and, in addition, taste and flavor that coffee originally has are lost. Accordingly, development of a new technology for producing decaffeinated coffee, which will replace the above-mentioned method, is desired.

Breeding of coffee plant is mainly performed by selection of a good variety by mating, propagation by seeds or grafting. However, it takes a long period of time for breeding by mating-selection and germination rate of coffee seeds decreases significantly depending on storage conditions. Propagation by grafting is effective in clone propagation of a good variety, however, securement of a large breeding station and renewal of rootstock are needed. Thereafter, tissue culture technology has been developed as a breeding technology for coffee plant replacing breeding by mating-selection (Berthouly and Etienne, Somatic Embryogenesis in Woody Plants, Vol. 5, Kluwer: 259-288, 1999). In addition, it has been possible to create a transgenic plant by genetic recombination technology recently, and several examples of transformation in coffee plant have been reported. As an example of the recent reports, there is a report on introduction of insect-resistant gene (Leroy et al. , Plant Cell Rep. 19: 382-389, 2000). As a new technology for producing decaffeinated coffee, molecular breeding of decaffeinated coffee utilizing genetic recombination technology has been theoretically proposed, however, there are no reports demonstrating it practically, yet.

An object of the present invention is to provide a new technology for molecular breeding of a coffee plant utilizing genetic recombination technology, specifically, to provide a process for producing coffee plant with low caffeine content by suppressing expression of genes coding for enzymes related to the caffeine biosynthetic pathway isolated from coffee by the antisense method or the RNAi (double-stranded RNA interference) method.

### DISCLOSURE OF THE INVENTION

The present invention relates to a process for producing a decaffeinated coffee plant by genetic recombination comprising;
a step for preparing an antisense sequence or an RNAi sequence of a gene coding for an enzyme related to the caffeine biosynthetic pathway and constructing an expression vector for transformation,
a step for introducing the obtained expression vector into Agrobacterium,
a step for infecting a cell division-activated tissue piece of a coffee plant or a callus or an adventitious embryo induced from a tissue piece of a coffee plant with the Agrobacterium to transform the tissue piece, the callus or the adventitious embryo and
a step for obtaining a transgenic coffee plant from a transformed tissue piece, a transformed callus or a transformed adventitious embryo.

In a coffee plant (coffee tree), 1,3,7-trimethylxanthine (caffeine) is biosynthesized from xanthosine as a starting material, which is a catabolite intermediate of adenine nucleotide and guanine nucleotide, via 7-mehylxanthosine, 7-methylxanthine and 3,7-dimethylxanthine (theobromine).

The above-mentioned enzyme related to the caffeine biosynthetic pathway can be any one of the enzymes that are xanthosine methyltransferase catalyzing methylation of xanthosine to form 7-mehylxanthosine, a nucleoside deribose enzyme catalyzing removal of rebose from 7-mehylxanthosine to form 7-methylxanthine, 7-methylxanthine methyltransferase catalyzing methylation of 7-methylxanthine to form 3,7-dimethylxanthine, and 3,7-dimethylxanthine methyltransferase catalyzing methylation of 3,7-dimethylxanthine to form 1,3,7-trimethylxanthine.

A cell division-activated leaf tissue piece is one partially forming callus in the state where cells mainly on the excised surface of the leaf are dividing actively. The cell division-activated leaf tissue piece can be obtained, for example, by pre-culturing a section tissue in a medium to which a phenylurea type cytokinin as a plant hormone is added. As the phenylurea type cytokinin, 4-CPPU (N-2-chloro-4-pyridyl-N-phenylurea) or TDZ (tydiazlon) is preferable.

Coffea genus to which a process of the present invention can be applied includes Coffea arabica species and Robusta species (Coffea canephora) that are in high demand on the global market as well as Coffea liberica species and Coffea dewevrei.

Accordingly, a decaffeinated coffee or a caffeine reduced coffee can be produced by obtaining coffee beans from the decaffeinated coffee plant obtained in accordance with the present invention.

### BRIEF DESCRIPTION OF DRAWINGS

Fig. 1 is a schematic illustration showing the expression vector for transformation and its modified vectors.

Fig. 2 indicates that the expression pattern of CaMXMT suppressed by the antisense method or the RNAi method in the obtained transformants was confirmed by the Reverse transcription (RT)-PCR method.

### BEST MODE FOR CARRYING OUT THE INVENTION

The present invention will be specifically described with reference to the following examples.

### Example 1 (Establishment of coffee tissue culture system)

In order to perform genetic recombination, a coffee tissue culture system was prepared with the following materials and methods.

### Materials:

Pot plants (Coffea arabica and Coffea canephora) which were grown in a greenhouse were used.

### Methods:

(1) A plant tissue of Coffea genus, preferably a new leaf, more preferably a first expanded leaf on the tip of a branch, was collected and sterilized. The sterilized tissue was cut into, for example, 2 to 20 mm square, preferably about 7 mm square, preferably in a clean bench.
   In the aforementioned sterilizing step, for example, the collected leaf was immersed in 70% ethanol for 1 minute, followed by a 2% aqueous solution of hypochlorous acid for 10 minutes. By this immersing operation, approximately 100% sterilization was achieved. This sterilizing solution and treatment conditions are adjusted as needed.
(2) A liquid or a solid culture medium containing a variety of nutritional components, for example MS medium (Murashige, Physiol. Plant 15: 473-497, 1962), B5 medium (Gamborg, Exp. Cell. Res. 50: 151-158, 1968), or a modified medium thereof, to which a plant hormone such as an adenine type cytokinin (BA: benzyladenine, 2ip: 2isopentenyl adenine), a phenylurea type cytokinin (4-CPPU: N-2-chloro-4-pyridyl-N-phenylurea or TDZ: tydiazlon) or 2,4-D: 2,4-dichlorophenoxyacetic acid, which is auxin, is added thereto. A combination of plant hormones is not limited to the aforementioned ones and is adjusted as needed. A modified medium is, for example, a modified MS medium in which the concentrations of only inorganic salts are reduced to half of the usual concentration (modified 1/2 MS: hereinafter abbreviated as m1/2MS). A concentration of the plant hormone is preferably 0.1 to 40 µM, more preferably 0.5 to 20 µM, most preferably 1 to 10 µM. To this medium, sucrose is added at preferably 10 to 100 g/L, more preferably 20 to 50 g/L and pH is adjusted to an optimal value, for example, 5.6 to 5.8, then a solidification agent is added thereto. (Hereinafter a solid medium means a medium which is solidified with Gellan Gum, and except otherwise stated, medium means a solid medium.) As the solidification agent, Gellan Gum is preferable and its amount to be added is 2 to 4 g/L. Then this medium is sterilized under high-pressure and high-temperature conditions. The sterilization conditions are preferably at 115 to 125°C for 10 to 30 minutes.
   In this embodiment, sucrose was added to a medium at 30 g/L and pH was adjusted to 5.7, then Gellan Gum was added at 3 g/L. The sterilizing conditions were high-pressure and high-temperature conditions at 121°C for 20 minutes.
(3) Subsequently, a sectioned leaf tissue piece of Coffea canephora was placed on a sterilized m1/2MS medium containing 1 to 40 µM of 2ip, especially 20 µM of 2ip, and cultured for 9 to 12 weeks (transplanted to a fresh medium of the same composition about every 3 weeks) to induce adventitious embryo. On the other hand, a sectioned leaf tissue piece of Coffea arabica was placed on a sterilized m1/2MS medium containing 1 µM of 4-CPPU and 5 µM of 2,4-D, and cultured for 3 to 9 weeks (transplanted to a fresh medium of the same composition about every 3 weeks) to induce callus. Except otherwise stated, the induced adventitious embryo and callus were propagated by transplanting them to a fresh medium of the same composition and used as a material for genetic recombination.

The conditions for induction culture of adventitious embryo or callus can be the same conditions as used for a conventional tissue culture of Coffea genus (for example, Berthouly and Michaux-Ferriere, Plant Cell, Tissue and Organ Culture 44: 169-176, 1996), and the temperature, for example, is 25 to 28°C. This culture is preferably performed in dark. In this example, culture was performed under the conditions at 25°C in dark. The culture is preferably performed continuously until cell division is induced macroscopically, namely, induction of a block of cultured cells are confirmed (for about 2 to 30 days).

### Example 2 (Construction of expression vector for transformation)

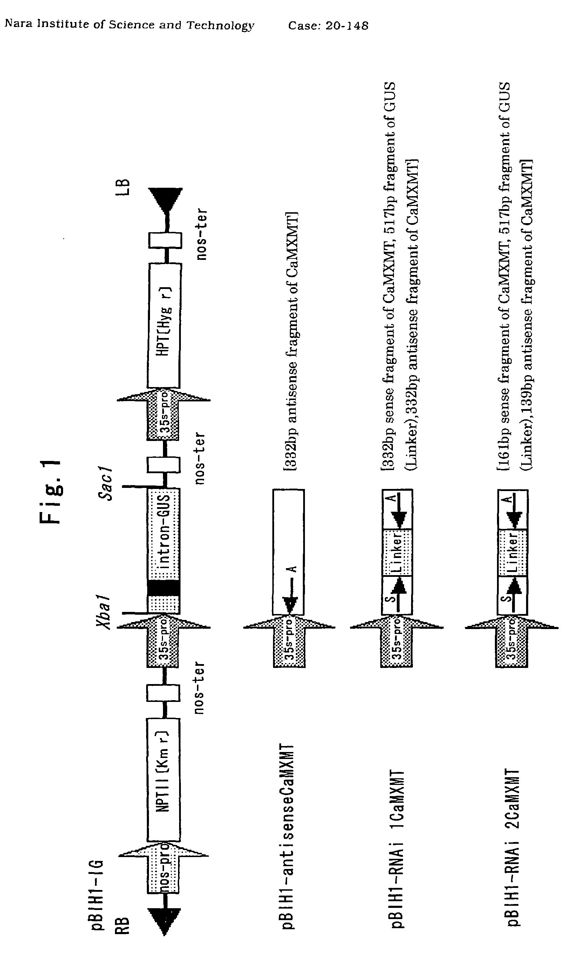

In this example, suppression of CaMXMT (gene coding for 7-methylxanthine methyltransferase (theobromine synthetic enzyme)) which the inventors have already isolated and analyzed (Ogawaet al. , J. Biol. Chem. 276: 8213-8218, 2001) is described. The cDNA corresponding to this gene, CaMXMT, has been registered as DDBJ/GenBank/EMBL accession number AB048794. There are various gene expression vectors which can be used as needed, and pIG121-Hm (pBIH1-IG) (Ohta et al. , Plant Cell Physiol. 31: 805-831, 1990) was used for this example. The gene expression vector pIG121-Hm (pBIH1-IG) is a vector having kanamycin-resistance gene regulated with NOS promoter, GUS (beta-glucuronidase) gene including intron regulated with cauliflower mosaic virus 35S promoter (CaMV35S) which is known as a promoter indicating strong transcriptional activity in plants and hygromycin-resistance gene. (See the top column in Fig. 1.) Therefore, these three genes are introduced into a plant by one operation. In this case, NOS terminator was inserted thereinto as a preferred terminator. A modified vector was constructed in order to regulate CaMXMT expression in accordance with the following procedures. (See the second column to the fourth column in Fig. 1.)
(1) A 332 bp fragment of CaMXMT (between 946 bp and 1277 bp) referred to as fragment A, a 161 bp fragment (between 1117 bp and 1277 bp) referred to as fragment B and a 139 bp fragment (between 1139 bp and 1277 bp) referred to as fragment C were amplified respectively. In addition, a 517 bp fragment of GUS (beta-glucuronidase) gene (between 3436 bp and 3952 bp) referred to as fragment D was amplified as a linker of RNAi construct.
   Incidentally, the gene fragments used for regulating CaMXMT expression and the gene fragment used as a linker of RNAi construct are not limited to the above regions, and they can be modified as needed, for example, including modified sequences such as base substitution of CaMXMT.
(2) GUS gene of pBIH1-IG including intron was digested with restriction enzymes (Xba I and Sac I) and fragment A was inserted as an antisense sequence into this site. Thus obtained modified vector is referred to as pBIH1-antisenseCaMXMT for the sake of convenience.
(3) GUS gene of pBIH1-IG including intron was digested with restriction enzymes (Xba I and Sac I) and fragment A as a sense sequence, fragment D as a linker sequence and fragment A as an antisense sequence were inserted into this site. Thus obtained modified vector is referred to as pBIH1-RNAi 1CaMXMT for the sake of convenience.
(4) GUS gene of pBIH1-IG including intron was digested with restriction enzymes (Xba I and Sac I) and fragment B as a sense sequence, fragment D as a linker sequence and fragment C as an antisense sequence were inserted into this site. Thus obtained modified vector is referred to as pBIH1-RNAi 2CaMXMT for the sake of convenience.

In Fig. 1, nos-pro stands for NOS promoter, Km r stands forkanamycin-resistance gene, 35S-pro stands for 35S promoter, intron-GUS stands for GUS including intron, nos-ter stands for NOS terminator, Hyg r stands for hygromycin-resistance gene, ←A stands for antisense sequence and S→ stands for sense sequence.

### Example 3 (Gene introduction with Agrobacterium)

Thus obtained expression vectors were used for gene introduction manipulation by the Agrobacterium method. Gene introduction by the Agrobacterium method can be performed according to a conventional method. Introduction of a foreign gene into a plant tissue can be performed by the binary vector method of Agrobacterium. There are various kinds of Agrobacterium strains and vectors and any of them can be used as needed. In this example, EHA101 (Hood et al., J. Bacteriol 168: 1291-1301, 1986), which is a strain of Agrobacterium tumefaciens and has been reported to be effective in genetic transformation in coffee plants (Hatanaka et al., Plant Cell Rep. 19: 106-110, 1999), was used. As a gene expression vector, the vectors constructed in Example 2, pBIH1-antisenseCaMXMT, pBIH1-RNAi 1CaMXMT and pBIH1-RNAi 2CaMXMT, which are modified vectors of pIG121-Hm (=pBIH1-IG), were used.

Before gene introduction into a Coffea genus plant, each vector is introduced into a strain of Agrobacterium: EHA101 by the electroporation method. Agrobacterium having one of the vectors is grown in a medium containing kanamycin and hygromycin, which are antibiotics, thus an Agrobacterium suspension is prepared.

In this Example, an example of gene introduction of Coffea arabica will be described. The procedures are as follows.
(1) The callus of Coffea arabica induced in accordance with the aforementioned Example 1 was cultured and proliferated under the same culture conditions (25°C in dark) as in Example 1.
(2) Agrobacterium having any one of the vectors was grown in a medium containing 100 mg/L of antibiotics kanamycin and hygromycin to prepare an Agrobacterium suspension having about 0.5 optical density (O.D.) at 600 nm. After the callus was immersed in the suspension for 30 minutes, it was blotted with sterilized paper filter to drain off the liquid. Thereafter, it was cultured for one day on a solid m1/2MS medium (containing 1 µM of 4-CPPU and 5 µM of 2,4-D) to which preferably 50 mg/L of acetosyringon (a gene expression inducer) was added.
(3) The callus cultured on a medium containing acetosyringon was washed with a liquid m1/2MS medium to which 300 mg/L of cefotaxime was added as a bacteriocidal agent for Agrobacterium, and cultured in a solidm1/2MS medium (containing 1 µM of 4-CPPU and 5 µM of 2, 4-D) to which 300 mg/L of cefotaxime and 50 mg/L of hygromycin as antibiotics were added. Subsequently, it was cultured for about 2 months on a medium to which 100 mg/L of antibiotics, but not cefotaxime, was added (transplanted to new medium of the same composition every 3 weeks), and after this period, transformants were obtained sequentially.

### Example 4 (Confirmation of introduced gene)

As mentioned above, the fact that the obtained transformants show antibiotic resistance indicates that, for example, hygromycin-resistance gene has been introduced and expressed. In addition, the genomic DNA was extracted, and introduction of the foreign gene has been confirmed by the PCR (polymerase chain reaction) method as well. For example, hygromycin-resistance gene was amplified with a primer pair of 5-GCGTGACCTATTGCATCTCC-3 and 5-TTCTACACAGCCATCGGTCC-3. PCR was performed for 30 cycles of heat denaturation at 94°C for 30 seconds, annealing at 58°C for 30 seconds and extension at 72°C for 30 seconds after heat denaturation at 94°C for 5 minutes. While an amplified DNA fragment was not observed in a non-transformant, a distinct band that shows the hygromycin-resistance gene fragment of 713 bp was confirmed in a transformant.

### Example 5 (Confirmation of CaMXMT expression)

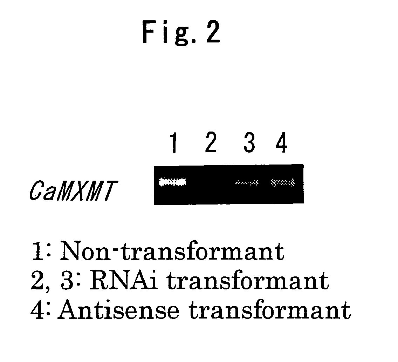

Regarding the obtained transformants, the expression pattern of CaMXMT, which is the gene suppressed by the antisense method or the RNAi method, was confirmed by the reverse transcription (RT)-PCR method. This is shown in Fig. 2. After extraction of total RNA, cDNA was synthesized using RNA PCR Kit (AMV) Ver.2.1 (Takara). CaMXMT was amplified with a primer pair of 5-TCCTACAATCTGGCTCTTGC-3 and 5-TGCTTTAATTTGTTCATGGGATC-3. PCR was performed for 24 cycles of heat denaturation at 94°C for 30 seconds, annealing at 58°C for 30 seconds and extension at 72°C for 1 minute.

As shown in Fig. 2, a distinct band was detected in a non-transformant (lane 1), whereas in the selected RNAi transformant (pBIH1-RNAi 1 CaMXMT introduction: lanes 2 and 3) and the antisense transformant (pBIH1-antisenseCaMXMT introduction: lane 4), the detected bands showed an apparent tendency of weak intensity. Among them, for example, the band in the RNAi transformant (lane 2) was hardly detected. This indicates that expression of CaMXMT in the selected transformant has been strongly suppressed.

### Example 6 (Quantitative determination of endogenous theobromine and caffeine contents by HPLC)

Regarding the obtained transformants, the contents of theobromine and caffeine which are endogenous purine alkaloids were measured by HPLC (High Performance Liquid Chromatography) . The purine alkaloids for analysis were obtained by the following hot-water extraction method. About 100 mg of fresh plant tissue, callus or the like was boiled at 80°C in 1 mL of ultra pure water for 20 minutes and this procedure was repeated twice. The extracted solution was filtered through a membrane filter of 0.2 µm of pore size to use it as an analysis sample. By using an HPLC system (Waters 600E), purine alkaloids were separated with a solution of methanol:water = 20:80, namely a 20% methanol solution. A Waters Puresil C18 column (4.6 mm×250 mm) was used. Extraction was performed for three independent samples, and analysis was performed three times for each sample. Then the mean value (standard deviation) of nine HPLC analytical values was calculated. The analytical results are shown in Table 1.

**Table 1**

| (Content of endogenous theobromine and caffeine in non-transformant and transformant) (mg/g dry weight) | | | | |
|---|---|---|---|---|
| Name of substance | non-transformant | RNAi transformant | | antisense transformant |
| | 1 | 2 | 3 | 4 |
| theobromine | 3.64(0.06) | 0.55(0.07) | 0.49(0.03) | 1.90(0.10) |
| caffeine | 1.06(0.06) | 0.00(0.00) | 0.00(0.00) | 0.14(0.01) |

In Table 1, numbers in parentheses denote standard deviation. Each sample number (1 to 4) corresponds to the lane number(1 to 4) in Fig 2.

As shown in Table 1, the contents of endogenous theobromine and caffeine in the transformants decrease significantly, especially in samples 2 and 3 of the RNAi transformants , caffeine was not detected. These results accord with those of Example 5 shown in Fig. 2 and indicate that, according to the present invention, expression of CaMXMT was strongly suppressed, therefore, a coffee transformant containing no caffeine was obtained.

## Claims

1. A process for producing a decaffeinated coffee plant by genetic recombination comprising;
a step for preparing an antisense sequence or an RNAi sequence of a gene coding for an enzyme related to the caffeine biosynthetic pathway and constructing an expression vector for transformation,
a step for introducing the obtained expression vector into Agrobacterium,
a step for infecting a cell division-activated tissue piece of a coffee plant or a callus or an adventitious embryo induced from a tissue piece of a coffee plant with the Agrobacterium to transform the tissue piece, the callus or the adventitious embryo and
a step for obtaining a transgenic coffee plant from a transformed tissue piece, a transformed callus or a transformed adventitious embryo.

2. A process for producing a decaffeinated coffee plant as claimed in claim 1, wherein the enzyme related to the caffeine biosynthetic pathway is xanthosine methyltransferase, a nucleoside deribose emzyme, a 7-methylxanthine methyltransferase or 3,7-dimethylxanthine methyltransferase.

3. A transformed coffee plant produced by the process as claimed in claim 1 or 2.
